# EUROPEAN PATENT APPLICATION

(11) **EP 4 275 728 A1**
(43) Date of publication of application: **15.11.2023**
(21) Application number: 22461546.8
(22) Date of filing: 10.05.2022
(51) Int. Cl.: A61M 15/00

(54) **AN ACTUATOR FOR AN INHALER**

(71) Applicant: Findair Sp. z o. o., 31-235 Krakow (PL)
(72) Inventor: MIKOSZ, Jacek, 31-162 Krakow (PL); CZYZ, Michal, 30-719 Krakow (PL); MIKOSZ, Tomasz, 32-091 Michalowice (PL)
(74) Representative: Kancelaria Eupatent.pl Sp. z.o.o

(57) **Abstract**

An actuator for an inhaler comprising: a body (10) comprising a mouthpiece (11) and a housing (12) for receiving a canister with a substance to be inhaled; a measuring module (20) with at least one sensor (21) wherein the measuring module is attachable to the body (10); wherein the body (10) comprises at least one through opening (13), wherein at least one sensor (21) of the measuring module (20) is positioned such that when the measuring module (20) is attached to the body (10), the sensor is in direct contact with the internal volume (19) of the body (10).

## Description

### TECHNICAL FIELD

The present invention relates to an actuator for an inhaler.

### BACKGROUND

An inhaler (also known as a puffer, pump, or allergy spray) is a medical device used for delivering medicines into the lungs through the work of a person's breathing. This allows medicines to be delivered to and absorbed in the lungs, which provides the ability for targeted medical treatment to this specific region of the body, as well as a reduction in the side effects of oral medications. Some of the common types of inhalers include metered-dose inhalers, dry powder inhalers, soft mist inhalers, and nebulizers. The most common type of inhaler is the pressurized metered-dose inhaler (MDI or pMDI) which comprises three main components - a metal canister, a plastic actuator, and a metering valve. The medication is typically stored in solution in the pressurized canister that contains a propellant or a suspension. The MDI canister is attached to the plastic, hand-operated actuator. On activation, the metered-dose inhaler releases a fixed dose of medication in aerosol form through the actuator and into a patient's lungs. These devices require significant coordination as a person must discharge the medication at or near the same time that they inhale it in order for the medication to be effective.

Most inhalers are not washable or sterilizable. That is why inhalers are designed to have a limited lifetime. Manufacturers of current pMDI aim for low costs. Manufacturing inhalers with additional components such as electronic or mechanical counters or radio transmitters is expensive and currently not very cost-effective.

Depending on the stage of illness, duration of use, or therapy, using an inhaler presents different challenges which change over time. In the early stages, the key problem is learning the inhalation technique and how to coordinate holding the inhaler with the right phase of inspiration. In the later stages, the key element is adherence to the assigned therapy as prescribed. There is also an increasingly important role for information on drug use in the clinical context. Such data can be used to assess disease progression and the effectiveness of prescribed therapy. Due to increasing technological capabilities, there is an emerging opportunity to measure data in an automated manner that would have previously had to be recorded manually.

### SUMMARY OF THE INVENTION

There is a need to provide an actuator with means for controlling the adherence of the medical substances by a user, in particular to facilitate learning of use of the inhaler or monitoring the use of the inhaler.

The invention relates to an actuator for an inhaler comprising: a body comprising a mouthpiece and a housing for receiving a canister with a substance to be inhaled; and a measuring module with at least one sensor wherein the measuring module is attachable to the body. The body comprises at least one through opening, wherein at least one sensor of the measuring module is positioned such that when the measuring module is attached to the body, the sensor is in direct contact with the internal volume of the body.

The at least one through opening can be located on a sidewall of the body, on a backwall of the body, on a mouthpiece, on a front wall of the body or on a bottom wall of the body.

The sensor can be a flowmeter, a microphone, a pressure sensor, a light sensor, a temperature sensor, a camera, a gas sensor, a volatile components (VOC) sensor or an alcohol sensor.

The measuring module may further comprise a display, a speaker, a video recorder, an NFC module, an RFID module, a press sensor such as a mechanical and/or electric button or a switch, a magnetic force sensor such as a Hall sensor, a humidity sensor, an accelerometer, a gyroscope, a magnetometer, a capacitive sensor such as a touch sensor, an ultrasound proximity sensor, an infrared proximity sensor, a resistive pressure sensor, a tensometer, or a piezoelectric sensor.

The attachment means may have a form of at least one of: a snap-fit connection, a form fit connection, a magnet, a groove and a bolt to be inserted into the groove.

The invention also relates to an inhaler comprising an actuator as described herein and a canister with a substance located in the housing of the body.

Therefore, the present invention provides an actuator for an inhaler that has the ability to have variable functions depending on the person being targeted, their experience with the disease and the inhaler as well as their involvement in the therapy. The variation of functions is provided by means of attachable modules with different electronic and mechanical circuits. This allows maintaining a low production cost of the actuator with the possibility of expanding the basic inhaler functionality to become an educational or a monitoring device.

The actuator for an inhaler according to the invention is suitable in particular for a metered-dose inhalers, but it may also be used with other types of inhalers such as dry powder inhalers, soft mist inhalers etc.

In view of that the at least one sensor of the measuring body is positioned such that when the measuring module is attached to the body, the sensor is in direct contact with the internal volume of the body, the sensor can accurately measure a parameter (such as a parameter related to flow of air while user inhales) within the internal volume of the body, which allows collecting precise measurements related to the inhalation process. The sensor measurement is less prone to inaccuracies resulting from external factors as compared to sensors of prior art devices which did not have access to the internal volume of the body.

These and other features, aspects and advantages of the invention will become better understood with reference to the following drawings, descriptions and claims.

### BRIEF DESCRIPTION OF DRAWINGS

The present invention is shown by means of preferable embodiments in a drawing, wherein:
Fig. 1 presents an embodiment of an actuator and a measuring module in an exploded view;
Figs. 2A-2C present schematically various configurations of the sensor with respect to the body.
Figs. 3A-3K present various other embodiments of actuators and measuring modules in an exploded view;
Fig. 3L presents an embodiment of an inhaler;
Figs. 4A-4B present an embodiment of the actuator with a covering plate in place of a measuring module;
Figs. 5A-5E present various embodiments of measuring modules.

### DETAILED DESCRIPTION

The following detailed description is of the best currently contemplated modes of carrying out the invention. The description is not to be taken in a limiting sense, but is made merely for the purpose of illustrating the general principles of the invention.

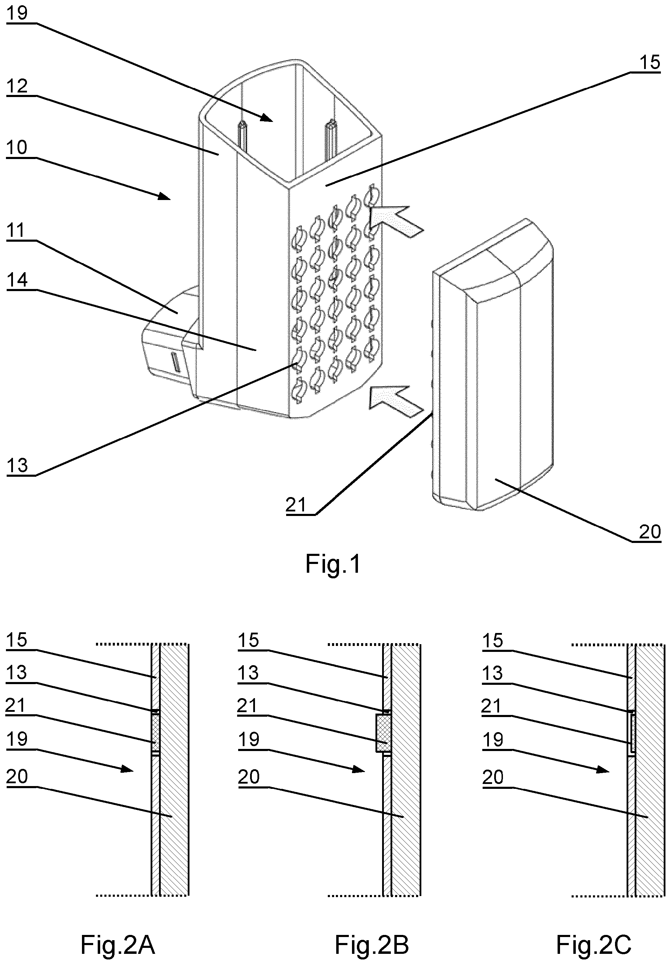

The actuator for an inhaler, as shown in illustrative embodiment of Fig. 1, has a body 10 comprising a mouthpiece 11 and a housing 12 for receiving a canister 51 with a substance to be inhaled. The actuator allows the patient to operate the inhaler. The canister 51, which is to be installed inside the housing 12, has a dosing device operable to allow the canister to be actuated to release a dose of the substance. The mouthpiece 11 is connected to the housing 12 and allows transferring the substance from the internal volume 19 of the body 10 to a user.

A measuring module 20 with at least one sensor 21 is attachable to the body 10. The body 10 has at least one through opening 13 for receiving the sensor 21 of the measuring module 20. At least one sensor 21 of the measuring module 20 is positioned such that when the measuring module 20 is attached to the body 10, then the sensor 21 is in direct contact with the internal volume 19 of the body 10.

The at least one sensor 21 may protrude above the surface of the measuring module such that when the measuring module 20 is attached to the body 10, a measuring surface of the sensor 21 is adjacent to the internal surface of the body 10, such as the internal surface of the back wall 15, as shown in Fig. 2A. Such alignment allows the sensor 21 to directly measure the conditions present inside the body 10. However other configurations of the sensor 21 alignment are also possible, for example the sensor 21 may protrude above the internal surface of the body 10, such that its measuring surface is positioned deeper inside the internal volume 19, as shown in fig. 2B. Yet alternatively, the sensor 21 may be aligned with the surface of the measuring module or only slightly protruding above its surface, such that the measuring surface of the sensor 21 is inside the opening 13, as shown in Fig. 2C.

The measuring module 20 allows to register the inhalation process. For example, the measuring module 20 may analyze the timing accuracy between the start of inhalation performed by the user and the time of initiation of a dose from the canister with the substance. Depending on the type of installed sensors, the measuring module 20 may also analyze the duration of the inhalation and the flow rate of the inhaled air. Taking this into account, it is possible to determine the level of adherence of the substance by the user. In particular, the user may be notified if the inhalation should be performed more intensively or whether the starting times of the inhalation and the actuation of the dosing mechanism are correct. As a result, the user may easily improve the performance of adherence of the medication.

The body 10 of the actuator may have more than one opening 13. In that case, only some of the openings 13 can be used when a particular measuring module 20 is attached to the actuator, while other openings can be used when a different measuring module 20 (for example, having a different functionality) is used.

The measuring module 20 is detachable from the actuator body 10 and can be mounted to different actuators. Similarly, measuring modules 20 with different functionalities (having different set of sensors) may be attached to a single actuator body 10 owned by the user. Therefore, the measuring module is replaceable.

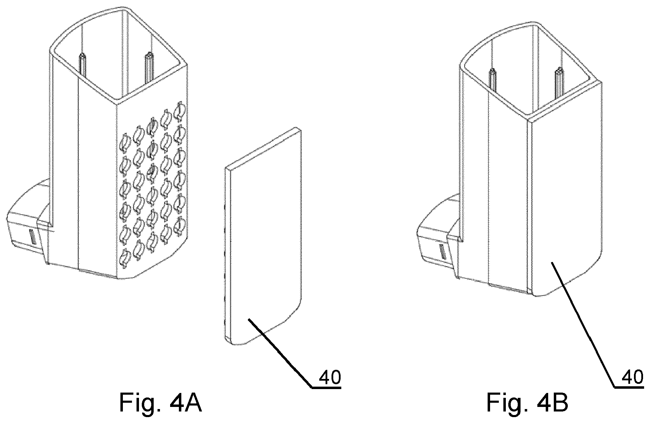

The actuator can be also used without any measuring module 20 attached - in that case, the openings 13 may be blocked or covered by a plate 40 (as shown in Figs. 4A-4B). The plate 40 can be attached to the actuator by the same type of attachment means 30 as used for the measuring module 20.

The means for attaching the measuring module 20 to the body 10 have different forms.

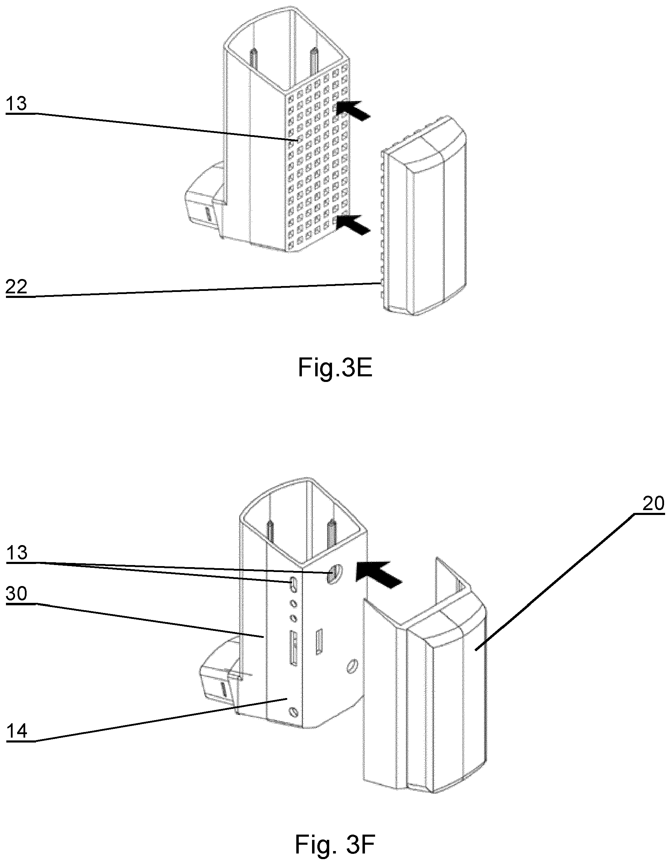

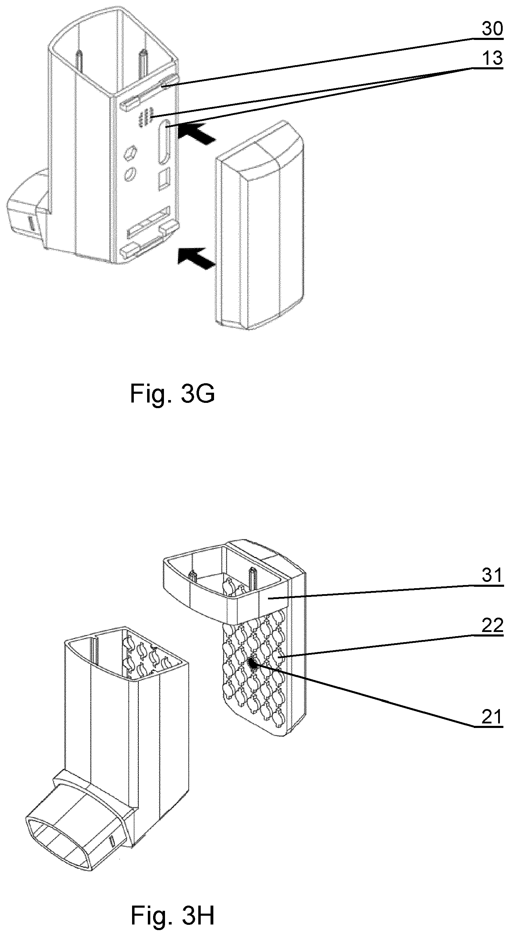

For example, ss presented in embodiments of Fig. 1, 3E and 3H, some of the openings 13 may be used as openings for accommodating the sensor 21, while other openings 13 may be used as attachment means 30 for attaching the measuring module 20 to the actuator. In that case, protrusions 22 present on the surface of the measuring module form a form fit connection with the openings 13 which are used as attachment means 30.

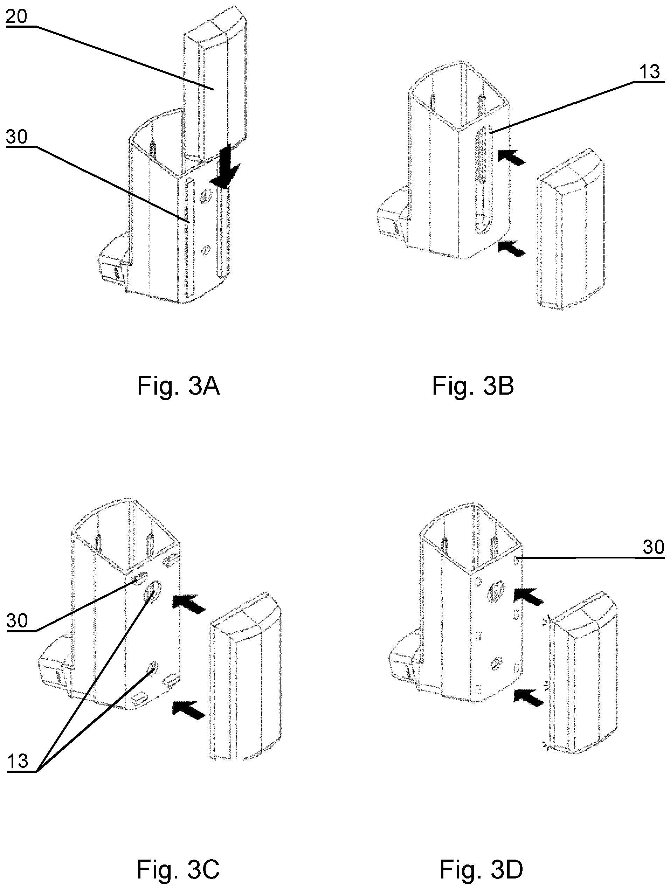

Alternatively, the attachment means 30 may have a form of magnets, as shown in Fig. 3D.

Alternatively, the attachment means 30 have a form of snap-fit connection, as shown in Figs. Figs. 3B, 3C, 3F and 3G. The attachment means 30 ma be located either on the sidewall 14 or on the backwall 15.

Fig. 3H presents a measuring module 20 with an extension rim 31, that once the measuring module 20 is attached to the body 10, makes the internal volume 19 higher. The extension rim 31 allows the actuator to be used with longer canisters which would otherwise excessively protrude above the actuator.

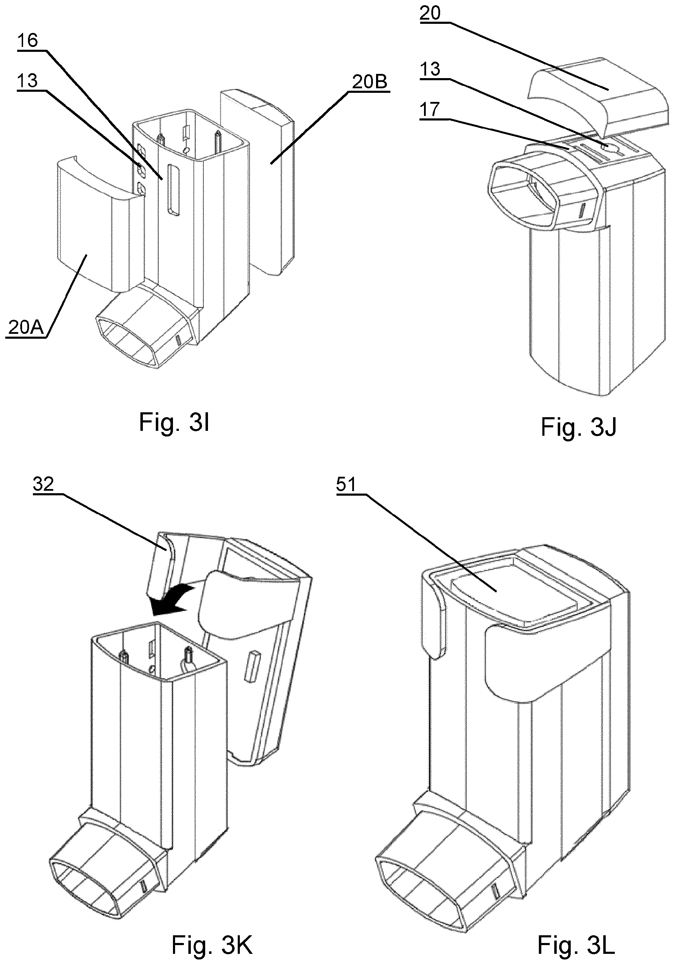

Fig. 3I presents an embodiment wherein a first measuring module 20A is connected at a front wall 16 of the body 10, while a second measuring module 20B is connected at the back wall.

Fig. 3J presents an embodiment wherein a measuring module 20 is connected to the top wall 17 of the body 10.

Fig. 3K presents an embodiment wherein a measuring module 20 is connected to the body 10 via a holder 32 with elastic arms to surround the body 10.

Fig. 3L presents an embodiment of Fig. 3K with a canister 51 with a medical substance to be inhaled installed therein, therefore forming an inhaler.

It is possible to provide multiple openings 13 each for a single sensor of the measuring module 20 or a few larger openings (as presented in figure 2A-2D, 2F, 2G), where multiple sensors may protrude through a single opening 13.

Depending on the functionality of the measuring module 20 and the type of sensors installed therein, the measuring of the conditions inside the housing may be performed by the sensor(s) 21 configured gain access to the internal volume 19 of the body via though openings 13 present at different locations within the housing 12 or at the mouthpiece 11. For example, the openings 13 for sensors 21 may be located on a backwall 15 of the body 10 (as shown in Figs. 1, 3A-3H, 3K), on a sidewall 14 of the body 10, on a mouthpiece 11, on a front wall 16 of the body 10 (as shown in Fig. 31) or on a bottom wall 17 of the body 1(as shown in Fig. 3J).

The sensor(s) 21 may be a flowmeter, a microphone, a pressure sensor, a light sensor, a temperature sensor, a camera, a gas sensor, a volatile components (VOC) sensor, an alcohol sensor.

Moreover, the measuring module may comprise further components, such as a display, a speaker, a video recorder, an NFC module, an RFID module, a press sensor such as a mechanical and/or electric button or a switch, a magnetic force sensor such as a Hall sensor, a humidity sensor, an accelerometer, a gyroscope, a magnetometer, a capacitive sensor such as a touch sensor, an ultrasound proximity sensor, an infrared proximity sensor, a resistive pressure sensor, a tensometer, a piezoelectric sensor.

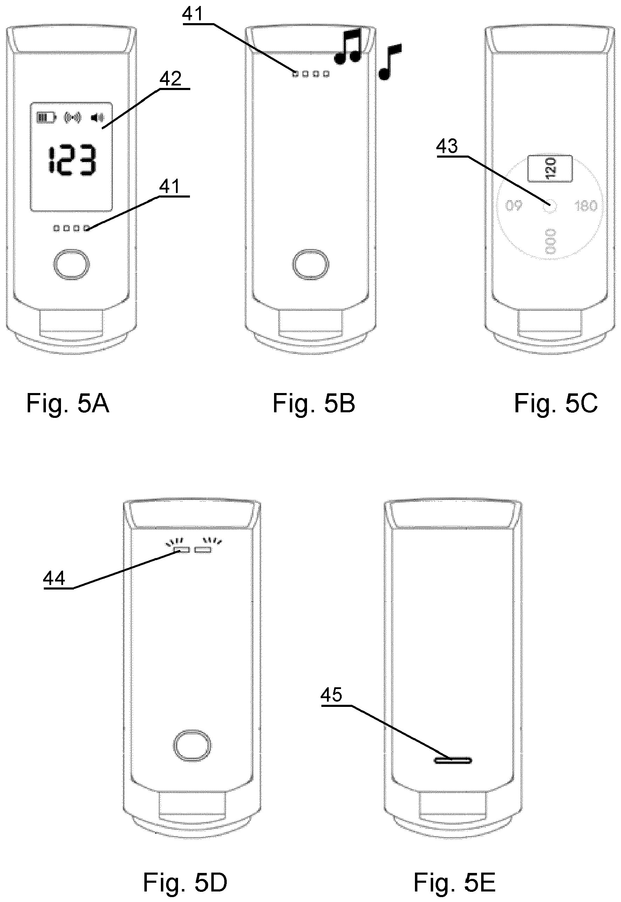

For example, an embodiment of the measuring module presented in Fig. 5A comprises a speaker 41 and a display 42 for informing the user about the status of the measuring module 20 such as battery level, number of inhaled/remaining doses etc.

For example, an embodiment of the measuring module 20 presented in Fig. 5B comprises a speaker 41 for sending audible information to the user.

For example, an embodiment of the measuring module 20 presented in Fig. 5C comprises a mechanical counter 43 of the inhaled doses.

For example, an embodiment of the measuring module 20 presented in Fig. 5D comprises a LED diodes indicator 44 for sending light signals to the user.

For example, an embodiment of the measuring module 20 presented in Fig. 5E comprises a USB port 45 for charging the measuring module 20 and/or transmitting collected data.

The measuring module may further comprise an NFC or an RFID module for identifying the actuator which is attached to the measuring module 20.

While the invention has been described with respect to a limited number of embodiments, it will be appreciated that many variations, modifications and other applications of the invention may be made. Therefore, the claimed invention as recited in the claims that follow is not limited to the embodiments described herein.

## Claims

1. An actuator for an inhaler comprising:
- a body (10) comprising a mouthpiece (11) and a housing (12) for receiving a canister with a substance to be inhaled; and
- a measuring module (20) with at least one sensor (21) wherein the measuring module is attachable to the body (10);
**characterized in that**
- the body (10) comprises at least one through opening (13), wherein at least one sensor (21) of the measuring module (20) is positioned such that when the measuring module (20) is attached to the body (10), the sensor is in direct contact with the internal volume (19) of the body (10).

2. The actuator according to claim 1 wherein the at least one through opening (13) is located on a sidewall (14) of the body (10).

3. The actuator according to claim 1 wherein the at least one through opening (13) is located on a backwall (15) of the body (10).

4. The actuator according to claim 1 wherein the at least one through opening (13) is located on a mouthpiece (11).

5. The actuator according to claim 1 wherein the at least one through opening is located on a front wall of the body.

6. The actuator according to claim 1 wherein the at least one through opening is located on a bottom wall of the body.

7. The actuator according to any of previous claims wherein the sensor (21) is a flowmeter, a microphone, a pressure sensor, a light sensor, a temperature sensor, a camera, a gas sensor, a volatile components (VOC) sensor or an alcohol sensor.

8. The actuator according to any of previous claims the measuring module (20) further comprises a display, a speaker, a video recorder, an NFC module, an RFID module, a press sensor such as a mechanical and/or electric button or a switch, a magnetic force sensor such as a Hall sensor, a humidity sensor, an accelerometer, a gyroscope, a magnetometer, a capacitive sensor such as a touch sensor, an ultrasound proximity sensor, an infrared proximity sensor, a resistive pressure sensor, a tensometer, or a piezoelectric sensor.

9. The actuator according to any of previous claims wherein the attachment means (30) have a form of at least one of: a snap-fit connection, a form fit connection, a magnet, a groove and a bolt to be inserted into the groove.

10. An inhaler comprising an actuator according to any of previous claims and a canister (51) with a substance located in the housing (12) of the body (10).
